# EUROPEAN PATENT SPECIFICATION

(11) **EP 4 178 381 B1**
(45) Date of publication and mention of the grant of the patent: **03.09.2025**
(21) Application number: 21745391.9
(22) Date of filing: 06.07.2021
(51) Int. Cl.: A24F 40/53, A61M 15/00, A61M 15/06

(54) **AEROSOL PROVISION SYSTEM**
AEROSOLBEREITSTELLUNGSSYSTEM
SYSTÈME DE FOURNITURE D'AÉROSOL

(30) Priority: 10.07.2020 GB 202010610
(43) Date of publication of application: 17.05.2023
(73) Proprietor: Nicoventures Trading Limited, London WC2R 3LA (GB)
(72) Inventor: ROSSER, Nicholas, London WC2R 3LA (GB); BRUTON, Connor, London WC2R 3LA (GB); NANDRA, Charanjit, London WC2R 3LA (GB); RUSHFORTH, David, London WC2R 3LA (GB); BAKER, Darryl, London WC2R 3LA (GB); KERSEY, Robert, London WC2R 3LA (GB); CROSIER, Mark, London WC2R 3LA (GB)
(74) Representative: Dehns
(86) International application number: PCT/GB2021/051716
(87) International publication number: WO 2022/008898

(56) References cited:
- WO-A1-2017/165413
- WO-A1-2017/205692
- WO-A1-2019/104227
- WO-A1-2020/037226
- GB-A- 2 542 270
- US-A1- 2012 048 266
- US-A1- 2014 345 633
- US-A1- 2016 089 508
- US-A1- 2016 331 027
- US-A1- 2019 335 817
- US-A1- 2020 022 416
- US-A1- 2020 178 616

## Description

### TECHNICAL FIELD

The present invention relates to an aerosol provision system.

### BACKGROUND

Electronic aerosol provision systems such as electronic cigarettes (e-cigarettes) generally contain an aerosol-generating material, such as a reservoir of a source liquid containing a formulation, typically including nicotine, or a solid material such as a tobacco-based product, from which an aerosol is generated for inhalation by a user, for example through heat vaporisation. Thus, an aerosol provision system will typically comprise an aerosol generator, e.g. a heating element, arranged to aerosolise a portion of aerosol-generating material to generate an aerosol in an aerosol generation region of an air channel through the aerosol provision system. As a user inhales on the device and electrical power is supplied to the aerosol generator, air is drawn into the device through one or more inlet holes and along the air channel to the aerosol generation region, where the air mixes with the vaporised aerosol generator and forms a condensation aerosol. The air drawn through the aerosol generation region continues along the air channel to a mouthpiece, carrying some of the aerosol with it, and out through the mouthpiece for inhalation by the user.

It is common for aerosol provision systems to comprise a modular assembly, often having two main functional parts, namely an aerosol provision device and disposable / replaceable consumable part. Typically the consumable will comprise the consumable aerosol-generating material and the aerosol generator (heating element), while the aerosol provision device part will comprise longer-life items, such as a rechargeable battery, device control circuitry and user interface features. The aerosol provision device may also be referred to as a reusable part or battery section and the consumable may also be referred to as a disposable part, cartridge or cartomiser.

The aerosol provision device and consumable are mechanically coupled together at an interface for use, for example using a screw thread, bayonet, latched or friction fit fixing. When the aerosol-generating material in a consumable has been exhausted, or the user wishes to switch to a different consumable having a different aerosol-generating material, the consumable may be removed from the aerosol provision device and a replacement consumable may be attached to the device in its place.

A potential drawback for aerosol provision systems is that there is no means to monitor the usage of the aerosol provision system by the user. This may lead to excessive use of the system by the user. Equally, the user may have little understanding of how they are using the system, such as when or how often they use the system.

Various approaches are described herein which seek to help address or mitigate some of the issues discussed above.

US2020/178616A1 discloses vaporizer device includes an inhalation nozzle, a container containing the vaporizable material; and an atomizer arranged for carrying out a single continual atomization process for producing an inhalable vapor. A dosage reminder system for the vaporizer device includes a control module, a reminder determination unit configured for detecting a parameter during the single continual atomization process of the atomizer and a reminder element arranged for providing a reminder effect under control of the control module when the parameter reaches to a predetermined threshold.

WO2020/037226A1 discloses a method for vaporizing a product of a plurality of different products including receiving, by a processor of a vaporizing device, a desired dosage amount that is indicative of an amount of a compound to release during one or more inhalation events. The method includes determining, by the processor, an occurrence of a current inhalation event and during the current inhalation event determining, by the processor, an inhalation pressure being applied to a container that contains the product; determining, by the processor, a predicted dosage that is indicative of a predicted amount of the compound that has been released in the vapor during the current inhalation event based on the inhalation pressure; and selectively adjusting, by the processor, a vaporizing temperature being applied to the product by the vaporizer based on the desired dosage and the predicted dosage.

US2014/345633A1 discloses an aerosol-generating system for oral or nasal delivery of a generated aerosol to a user is provided, including a heater element configured to heat an aerosol-forming substrate to generate an aerosol; a power source; a controller configured to control operation of the heater element, the controller including or being connected to a means to detect a change in air flow past the heater element; first data storage means for recording detected changes in airflow past the heater element and data relating to the operation of the heater element; second data storage means including a database relating changes in airflow and data relating to the operation of the heater element to the properties of the aerosol delivered to the user; and an indication means coupled to the second data storage means for indicating to the user a property of the aerosol delivered to the user.

US2012/048266A1 discloses an apparatus including a first cartridge, a sensor, and a controller. The first cartridge can include a first release device configured to release a first substance into a housing. The controller can be configured to receive data from the sensor. The controller can determine an amount of first substance released by the first cartridge based on the data. The first release device can be controlled based on the determined amount of first substance.

WO2017/165413A1 discloses a system for administering a dosage of an inhalable product to a user including a vaporizing device for converting an active pharmaceutical ingredient (API) into the inhalable product to treat an affliction. The vaporizing device includes a communication element to send/receive at least one piece of dosage data to/from a portable electronic device, the dosage data corresponding to one or more properties of the dosage. The system also includes at least one processor and a memory. Memory includes machine-readable instructions that, when executed by the at least one processor, cause the system to receive an indicator corresponding to the API utilized by the vaporizing device, to determine the dosage for the API utilized by the vaporizing device, to transmit, to the user, instructions for administering the dosage, and to request feedback from the user regarding the efficacy of the dosage.

US2020/022416A1 discloses a vaporizing article, comprises a vaporizer drive circuit; one or more memories configured to store a percentage of at least one constituent in an inhalation media, one or more compensation values for at least one compensation category for the at least one constituent, and instructions; and a control circuit comprising a processor coupled with the one or more memories configured to run the instructions, the instructions configured to cause the processor to: receive a dose target for a constituent; determine whether to perform compensation for an inhalation media dose in order to ensure the dose target is met; when compensation is to be performed, determine the properly compensated inhalation media dose based on an associated compensation value for the constituent; and control the vaporizer drive circuit so as to dispense a compensated dose of the inhalation media.

US2019/335817A1 discloses an inhalation device for inhaling a vaporized substance that includes metering capabilities to inform a user when a particular amount of substance has been consumed. The inhalation device can include a sensor that senses the vaporized substance and a processor that utilizes data from the sensor to meter the amount consumed. The inhalation device can also define a session, which is a time in which a user can consume a particular amount. During the session, a user can start and stop inhaling and resume inhaling. When the user stops inhaling the inhalation device will halt vapor production and will resume production when the user resumes inhaling.

GB2542270A discloses An electronic cigarette vaporiser system including a cartridge designed to provide a liquid or other substance for the electronic vaporiser system, the cartridge including a chip that stores data related to the batch number of the substance stored in the cartridge, and the cartridge being adapted to be inserted into or form an integral part of the electronic vaporiser system. The electronic vaporiser system reads the data from the chip and compares that data with stored data and, depending on the result of that comparison, either prevents or permits use of that substance. The data stored can relate to batch number, shelf life, date of manufacture or other identifying features. The vaporiser or storage case can deny operation of the fluid transfer system on the cartridge if the ID is denied or flagged by the remote server.

US 2016/089508A1 discloses a vapour inhalation device, and WO2017/205692A1 discloses control of an electronic vaporiser.

### SUMMARY

The invention is defined in the appended claims.

In accordance with some embodiments described herein, there is provided an aerosol provision system. The aerosol provision system comprises an aerosol generator configured to aerosolize an aerosol-generating material. The aerosol provision system also comprises a sensor configured to detect an inhalation on the aerosol provision system by a user of the aerosol provision system, and output corresponding inhalation detection signals to control circuitry. The control circuitry is configured to determine an indication of an amount of an ingredient delivered from the aerosol generating material to the user during the inhalation based on the inhalation detection signals, compare the indication of the amount of the ingredient delivered to a threshold, and provide a notification to the user based on the comparison between the indication of the amount of the ingredient delivered to the threshold.

The comparison of the indication of the amount of the ingredient delivered to the threshold may be performed based on an indication of the amount of the ingredient delivered from the aerosol-generating material to the user during each inhalation in a predetermined period, for example an hour, 24 hours or a week.

The comparison of the indication of the amount of the ingredient delivered to the threshold may be performed after each inhalation. The comparison of the indication of the amount of the ingredient delivered to the threshold may be performed during each inhalation, for example at a predetermined interval during the inhalation.

The determination of the indication of the amount of the ingredient delivered from the aerosol-generating material to the user may be based on a concentration of the ingredient in the aerosol-generating material. The ingredient may be nicotine, caffeine, taurine, theine, a vitamin, melatonin, or a cannabinoid.

The notification may be provided to the user when the indication of the amount of the ingredient delivered exceeds the threshold.

The notification may be provided to the user based on the comparison between the indication of the amount of the ingredient delivered to a plurality of thresholds.

According to one aspect of the invention, the control circuitry is configured to determine default user behaviour based on the indication of the amount of the ingredient delivered from the aerosol-generating material to the user. The default user behaviour may determined over an inhalation. The control circuitry may be configured to determine a time between the inhalation and a next inhalation based on the inhalation detection signals, and determine default user behaviour based on the time between the inhalation and the next inhalation. The default user behaviour may be determined over a plurality of inhalations, and a time between each of the plurality of inhalations is less than a predetermined time. The default user behaviour may be determined over a rolling time period. The threshold is determined based on the determined default user behaviour.

The notification may be provided on the aerosol provision system and/or an application on a remote device. The notification may be a haptic notification. A parameter of the haptic notification may be adjustable by the user. The notification may be configurable by the user.

In accordance with some embodiments described herein, there is provided a system comprising an aerosol provision system configured to generate aerosol from an aerosol-generating material. The system also comprises a computer configured to determine an indication of an amount of an ingredient delivered from the aerosol-generating material aerosolized by an aerosol generator to the user during the inhalation based on inhalation detection signals received from a sensor of the aerosol provision system, compare the indication of the amount of the ingredient to a threshold, and provide a notification to the user based on the comparison between the indication of the amount of the ingredient delivered to the threshold.

In accordance with some embodiments described herein, there is provided a method of operating an aerosol provision system comprising receiving inhalation detection signals from a sensor configured to detect an inhalation on the aerosol provision system by a user of aerosol provision system, determining an indication of an amount of an ingredient delivered from an aerosol-generating material aerosolized by an aerosol generator to the user during the inhalation based on the inhalation detection signals, comparing the indication of the amount of the ingredient to a threshold, and providing a notification to the user based on the comparison between the indication of the amount of the ingredient delivered to the threshold. There is also provided a computer readable storage medium comprising instructions which, when executed by a processor, performs the above method.

These aspects and other aspects will be apparent from the following detailed description. In this regard, particular sections of the description are not to be read in isolation from other sections.

### BRIEF DESCRIPTION OF DRAWINGS

Embodiments of the invention will now be described, by way of example only, with reference to accompanying drawings, in which:
Figures 1 and 2 are schematic diagrams of an aerosol provision system;
Figures 3A to 3C illustrate graphs of inhalation detection signal output by the sensor against time;
Figure 4 illustrates a system comprising an aerosol provision system and a computer;
Figure 5 is a flow chart of a method of operating an aerosol provision system.

### DETAILED DESCRIPTION

Aspects and features of certain examples and embodiments are discussed / described herein. Some aspects and features of certain examples and embodiments may be implemented conventionally and these are not discussed / described in detail in the interests of brevity. It will thus be appreciated that aspects and features of articles and systems discussed herein which are not described in detail may be implemented in accordance with any conventional techniques for implementing such aspects and features.

The present disclosure relates to aerosol provision systems, which may also be referred to as aerosol provision systems, such as e-cigarettes. Throughout the following description the term "e-cigarette" or "electronic cigarette" may sometimes be used, but it will be appreciated this term may be used interchangeably with aerosol provision system and electronic aerosol provision system.

As noted above, aerosol provision systems (e-cigarettes) often comprise a modular assembly including both a reusable part (aerosol provision device) and a replaceable (disposable) cartridge part, referred to as a consumable. Systems conforming to this type of two-part modular configuration may generally be referred to as two-part systems or devices. It is also common for electronic cigarettes to have a generally elongate shape. For the sake of providing a concrete example, certain embodiments of the disclosure described herein comprise this kind of generally elongate two-part system employing disposable cartridges. However, it will be appreciated the underlying principles described herein may equally be adopted for other electronic cigarette configurations, for example modular systems comprising more than two parts, as devices conforming to other overall shapes, for example based on so-called box-mod high performance devices that typically have a more boxy shape.

As described above, the present disclosure relates to (but it not limited to) aerosol provision devices and corresponding aerosol provision systems, such as e-cigarettes and electronic cigarettes.

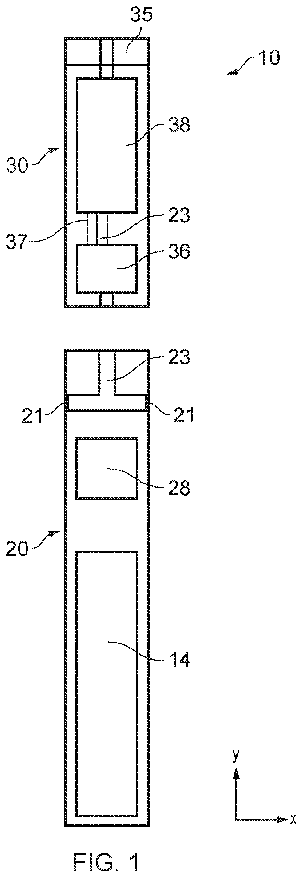

Figure 1 is a highly schematic diagram (not to scale) of an example aerosol provision system 10, such as an e-cigarette, to which embodiments are applicable. The aerosol provision system has a generally cylindrical shape, extending along a longitudinal or y axis as indicated by the axes (although aspects of the invention are applicable to e-cigarettes configured in other shapes and arrangements), and comprises two main components, namely an aerosol provision device 20 and a consumable 30.

The consumable 30 is an article comprising or consisting of aerosol-generating material 38, part or all of which is intended to be consumed during use by a user. A consumable 30 may comprise one or more other components, such as an aerosol-generating material storage area, an aerosol-generating material transfer component 37, an aerosol generation area, a housing, a wrapper, a mouthpiece 35, a filter and/or an aerosol-modifying agent.

A consumable 30 may also comprise an aerosol generator 36, such as a heating element, that emits heat to cause the aerosol-generating material 38 to generate aerosol in use. The aerosol generator 36 may, for example, comprise combustible material, a material heatable by electrical conduction, or a susceptor. It should be noted that it is possible for the aerosol generator 36 to be part of the aerosol provision device 20 and the consumable 30 then may comprise the aerosol-generating material storage area for the aerosol-generating material 38 such that, when the consumable 30 is coupled with the aerosol provision device 20, the aerosol-generating material 38 can be transferred to the aerosol generator 36.

The aerosol-generating material 38 is a material that is capable of generating aerosol, for example when heated, irradiated or energized in any other way. The aerosol-generating material 38 may, for example, be in the form of a solid, liquid or gel which may or may not contain an active substance and/or flavourants. In some embodiments, the aerosol-generating material 38 may comprise an "amorphous solid", which may alternatively be referred to as a "monolithic solid" (i.e. non-fibrous). In some embodiments, the amorphous solid may be a dried gel. The amorphous solid is a solid material that may retain some fluid, such as liquid, within it. In some embodiments, the aerosol-generating material may for example comprise from about 50wt%, 60wt% or 70wt% of amorphous solid, to about 90wt%, 95wt% or 100wt% of amorphous solid.

The aerosol-generating material 38 comprises one or more ingredients, such as one or more active substances and/or flavourants, one or more aerosol-former materials, and optionally one or more other functional materials such as pH regulators, colouring agents, preservatives, binders, fillers, stabilizers, and/or antioxidants.

The active substance as used herein may be a physiologically active material, which is a material intended to achieve or enhance a physiological response. The active substance may for example be selected from nutraceuticals, nootropics, psychoactives. The active substance may be naturally occurring or synthetically obtained. The active substance may comprise for example nicotine, caffeine, taurine, theine, vitamins such as B6 or B12 or C, melatonin, cannabinoids, or constituents, derivatives, or combinations thereof. The active substance may comprise one or more constituents, derivatives or extracts of tobacco, cannabis or another botanical.

In some embodiments, the active substance comprises nicotine. In some embodiments, the active substance comprises caffeine, melatonin or vitamin B12.

The aerosol provision device 20 includes a power source 14, such as a battery, configured to supply electrical power to the aerosol generator 36. The power source 14 in this example is rechargeable and may be of a conventional type, for example of the kind normally used in electronic cigarettes and other applications requiring provision of relatively high currents over relatively short periods. The battery 14 may be recharged through the charging port (not illustrated), which may, for example, comprise a USB connector.

The aerosol provision device 20 includes control circuitry 28 configured to monitor and/or control the operation of the aerosol provision system 10 and provide conventional operating functions in line with the established techniques for controlling aerosol provision systems such as electronic cigarettes. The control circuitry (processor circuitry) 28 may be considered to logically comprise various sub-units / circuitry elements associated with different aspects of the electronic cigarette's operation. For example, depending on the functionality provided in different implementations, the control circuitry 28 may comprises power source control circuitry for controlling the supply of electrical power from the power source 14 to the aerosol generator 36, user programming circuitry for establishing configuration settings (e.g. user-defined power settings) in response to user input, as well as other functional units / circuitry associated functionality in accordance with the principles described herein and conventional operating aspects of electronic cigarettes. It will be appreciated the functionality of the control circuitry 28 can be provided in various different ways, for example using one or more suitably programmed programmable computer(s) and / or one or more suitably configured application-specific integrated circuit(s) / circuitry / chip(s) / chipset(s) configured to provide the desired functionality.

The aerosol provision device 20 illustrated in Figure 1 includes one or more air inlets 21. In use, as a user inhales on the mouthpiece 35, air is drawn into the aerosol provision device 20 through the air inlets 21 and along an air channel 23 to the aerosol generator 36, where the air mixes with the vaporised aerosol-generating material 38 and forms a condensation aerosol. The air drawn through the aerosol generator 36 continues along the air channel 23 to a mouthpiece 35, carrying some of the aerosol with it, and out through the mouthpiece 35 for inhalation by the user. It will be appreciated that the one or more air inlets may be provided on the consumable 30 such that the air channel 23 is entirely contained within the consumable 30, or the aerosol provision device 20 and the consumable 30 may each comprise at least one air inlet 21 and a portion of the air channel 23.

By way of a concrete example, the consumable 30 comprises a housing (formed, e.g. from a plastics material), a reservoir formed within the housing for containing the aerosol-generating material 38 (which in this example may be a liquid which may or may not contain nicotine), an aerosol-generating material transfer component 37 (which in this example is a wick formed of e.g., glass or cotton fibres, or a ceramic material configured to transport the liquid from the reservoir using capillary action), an aerosol generating area, and a mouthpiece 35. Although not shown, a filter and/or aerosol modifying agent (such as a flavour imparting material) may be located in, or in proximity to, the mouthpiece 35. The consumable of this example comprises a heater element formed from an electrically resistive material (such as NiCr8020) spirally wrapped around the aerosol-generating material transfer component 37, and located in the air channel 23. The area around the heating element and wick combination is the aerosol generating area of the consumable 30. The consumable comprises suitable electrical contacts for coupling to electrical contacts provided on the aerosol provision device 20, such that electrical power may be supplied directly to the heater element.

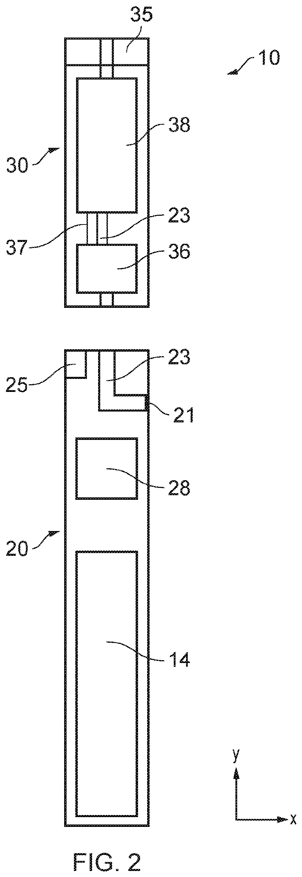

Figure 2 is a schematic diagram of a further example of an aerosol provision system 10, where the same reference signs have been used for like elements between the aerosol provision system 10 illustrated in Figure 1 and the aerosol provision system 10 illustrated in Figure 2.

The aerosol provision system 10 in Figure 2 comprises a sensor 25 configured to detect an inhalation on the aerosol provision system 10 by a user of the aerosol provision system 10. For example, the sensor 25 may be a flow sensor, a microphone, a pressure sensor, light sensor, touch sensor, accelerometer, gyroscope, or any other type of sensor suitable for directly or indirectly detecting or inferring an inhalation on the aerosol provision system 10 by a user of the aerosol provision system 10. Although the sensor 25 illustrated in Figure 2 is part of the aerosol provision device 20, this is not essential. In other embodiments the sensor 25 may be part of the consumable 30.

The sensor 25 may be configured to detect an inhalation based on the flow of air into one or more of the air inlets 21, or in the air channel 23 through the aerosol provision system 10. Alternative, the sensor may include a pressure sensor or light sensor on the mouthpiece 35 configured to detect when the user's lips are placed around the mouthpiece 35, or a pressure sensor or light sensor located on the aerosol provision device 20 to detect when the user places their hand around the aerosol provision device 20.

In some examples there is more than one sensor 25. For example, there may be a sensor 25 located proximate to an air inlet and a sensor 25 proximate to a portion of the air channel 23, the aerosol generator 36 and/or the mouthpiece 35 as described above. Accordingly, each sensor is configured to detect an inhalation on the aerosol provision system 10. Where there is more than one sensor 25, this can comprise more than one type of sensor, and/or multiple sensors of the same type.

In response to detecting an inhalation on the aerosol provision system 10 by a user of the system, the sensor 25 is configured to output corresponding inhalation detection signals to the control circuitry 28. In some embodiments, the sensor 25 is configured to continuously output inhalation detection signals, or to output inhalation detection signals periodically, such as every 0.01 seconds, every 0.1 seconds, or every 1 second. If the sensor 25 outputs inhalation detection signals periodically, then in some implementations the period between the output of subsequent inhalation detection signals may be set equal to or less than the average, or a typical length of an user inhalation (e.g. between 2 to 5 seconds) so as to ensure that an inhalation is not missed. In each case, the inhalation detection signals change when an inhalation on the system is detected by the sensor 25. For example, the inhalation detection signals could be a binary indication of whether an inhalation on the system is detected or not, for example a "1" to indicate an inhalation has been detected and a "0" to indicate that an inhalation has not been detected. Alternatively, the inhalation detection signals could correspond to an inhalation level or strength detected by the sensor 25. In other words, the inhalation detection signals could provide an indication of the draw strength detected by the sensor 25. For example, if the sensor 25 is a microphone or a flow sensor, the inhalation detection signals could provide an indication of the air speed or mass flow through the aerosol provision system 10, thereby providing an indication of the magnitude or strength of the inhalation taken by the user. In some embodiments, the inhalation detection signals correspond to the signals detected by the sensor 25. In other words, the inhalation detection signals represent the raw output from the sensor 25 without any filtering or processing applied by the sensor 25.

The inhalation detection signals could be set to 0 when an inhalation has not been detected by the sensor 25 and correspond to the inhalation level or strength detected by the sensor 25 when an inhalation has been detected. In some embodiments, the sensor 25 is configured to only output inhalation detection signals when an inhalation has been detected. In other words, the sensor 25 is configured output inhalation detection in response to detecting an inhalation on the system and the sensor 25 is configured to stop outputting inhalation detection signals when the inhalation is no longer detected by the sensor 25.

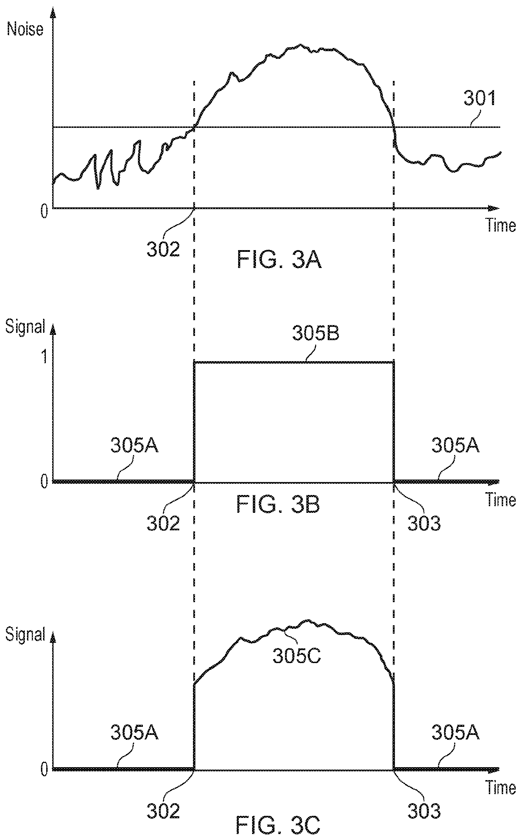

Figures 3A to 3C illustrate graphs of inhalation detection signal output by the sensor 25 against time. In the example illustrated in Figure 3A, the sensor 25 outputs inhalation detection signals continuously, and an inhalation being detected by the sensor 25 corresponds to the period when the inhalation detection signals are greater than a detection threshold 301. In the example illustrated in Figure 3B, until time point 302, which corresponds to the time point at which the sensor 25 detects an inhalation by the user on the aerosol provision system 10, the inhalation detection signal 305A output by the sensor 25 is "0". This may either represent no inhalation detection signal being output, or an inhalation detection signal being output with a value equal to "0". Between time point 301 and 302, which corresponds to the times during which the sensor 25 detects an inhalation by the user on the aerosol provision system 10, the inhalation detection signal 305B output by the sensor 25 is "1". In other words, the sensor 25 outputs an inhalation detection signal to indicate that an inhalation is detected. After time point 303, the inhalation detection signal 305A output by the sensor 25 is "0", indicating that the sensor 25 no longer detects an inhalation. As set out above, this may either represent no inhalation detection signal being output, or an inhalation detection signal being output with a value equal to "0". In the example illustrated in Figure 3C the inhalation detection signals 305A are set to "0" when an inhalation is not detected by the sensor 25, and the inhalation detection signal 305C corresponds to the signal recorded by the sensor 25 when the sensor 25 detects an inhalation.

As described above, the sensor 25 is configured to output the inhalation detection signals to the control circuitry 28. In response to receiving the inhalation detection signals, the control circuitry 28 is configured to determine an indication of an amount of an ingredient delivered from the aerosol-generating material to the user during the inhalation based on the inhalation detection signals. As it will be appreciated, the amount of aerosol, and by extension the amount of aerosol-generating material 38, delivered to the user during an inhalation will vary based on various factors, such as the duration of the inhalation, the type of aerosol-generating material 38 aerosolised, the temperature of aerosol generator 36, the amount of electrical power delivered to the aerosol generator 36 and the speed or mass flow of the air through the aerosol provision system 10. As described above, the aerosol-generating material 38 comprises one or more ingredients. Accordingly, the amount of each of the one or more ingredients delivered from the aerosol-generating material 38 to the user during the inhalation will also vary based on the amount of aerosol delivered to the user during the inhalation. Accordingly, the control circuitry is configured to use the inhalation detection signals received from the sensor 25 in order to determine an indication of an amount of an ingredient delivered from the aerosol-generating material to the user during the inhalation based on the inhalation detection signals.

In some embodiments, the control circuitry 28 is configured to determine a duration of the inhalation based on the inhalation detection signals received from the sensor 25, which can then be used in the determination of the indication of the amount of an ingredient delivered from the aerosol-generating material 38 to the user during the inhalation. In other words, the control circuitry 28 may be configured to determine the elapsed time for an inhalation based on the inhalation detection signals received from the sensor 25. As set out above, the sensor 25 may be configured to continuously or periodically output inhalation detection signals to the control circuitry, and the control circuitry is configured to use the change in these signals described above to determine a duration of an inhalation, for example by starting an inhalation timer when the inhalation detection signals change a first time and stop the inhalation timer when the inhalation detection signals change a second time. The control circuitry 28 may be configured to start the inhalation timer when the first non-zero inhalation detection signal is received, or when the first inhalation detection signal indicative of an inhalation being detected by the sensor 25 is received, such as at time 302 in Figures 3B and 3C respectively. The control circuitry 28 may then be configured to stop the inhalation timer when the next zero value inhalation detection signal is received, or when the next inhalation detection signal indicative of the sensor 25 no longer detecting an inhalation is received, such as at time 303 in Figures 3A-3C. Using Figures 3A-3C as an example, the duration of inhalation determined by the control circuitry 28 is the elapsed time between the time points 302 and 303.

Alternatively, the duration of an inhalation may be determined based on information contained within the inhalation detection signals, such as a time stamp associated with each inhalation detection signal. For example, in the case illustrated in Figure 3B or 3C, the control circuitry 28 is configured to use the timestamp of a first non-zero inhalation detection signal (at time 302) received and the timestamp of the next zero value inhalation detection signal received (at lime 303) to determine the duration of the inhalation. Alternatively, in the example illustrated in Figure 3A, the control circuitry 28 is configured to use the timestamp of the first inhalation detection signal exceeding the detection threshold 301 received from the sensor 25, corresponding to time point 302 in the Figure 3A, and the timestamp of the next inhalation detection signal indicative not exceeding the detection threshold received from the sensor, corresponding to time point 303 in Figure 3A, to determine the duration of the inhalation.

In some embodiments, the control circuitry 28 is configured to determine a time between an inhalation and the next inhalation based on the inhalation detection signals. In other words, the control circuitry 28 is configured to determine the elapsed time between an inhalation and the inhalation that follows it. This can be achieved using the same techniques as described above with respect to determining the duration of an inhalation, such as using a timer, information contained within the inhalation detection signals or the period of output of the inhalation detection signals.

The control circuitry 28 may also be configured to determine one or more operational parameters of the aerosol provision system 10 which can be used in the determination of the indication of the amount of an ingredient delivered from the aerosol-generating material 38 to the user during the inhalation. For example, the operation parameter may be an amount of electrical power supplied to the aerosol generator 36 by the power source 14. The control circuitry 28 is then configured to determine the indication of the amount of the ingredient delivered from the aerosol-generating material 38 to the user during the inhalation based on an indication of the amount of electrical power supplied to the aerosol generator 36 by the power source 14 during the inhalation. For example, the indication of the amount of electrical power supplied could be the amount of power delivered or a voltage and/or current supplied to the aerosol generator 36 during the inhalation, or could be a power setting for the aerosol generator 36 during the inhalation, such as an integer between 1 and 10 or "1" for low, "2" for medium and "3" for high. The amount of aerosol generated by the aerosol generator 36 during an inhalation will vary depending on the amount of electrical power supplied to the aerosol generator 36, and therefore a more accurate determination of the indication of the amount of the ingredient delivered from the aerosol-generating material 38 to the user during the inhalation can be achieved by considered the amount of electrical power in the calculation.

Alternatively or in addition, control circuitry 28 may determine one or more other operational parameters of the aerosol provision system 10, such as an amount of charge in the power source 14, a temperature of the aerosol generator 36 or a temperature proximate to the aerosol generator 36, an amount and/or speed of airflow through the aerosol provision system 10, indications of which are then used to determine the indication of the amount of the ingredient delivered from the aerosol-generating material 38 to the user during the inhalation. The operational parameter may change or vary during an inhalation, for example a decrease in the amount of charge in the power source 14 or an increase in the temperature of the aerosol generator 36. The determination of the operational parameter by the control circuitry 28 may therefore correspond to a maximum value, a minimum value or an average, model or median value of the operational parameter during the inhalation. Equally, the indication of the operational parameter may represent one or more of a value for the operational parameter at the start of the inhalation, a value for the operational parameter at the end of the inhalation, a maximum value of the operation parameter during the inhalation, a minimum value of the operation parameter during the inhalation and an average, model and/or median value for the operational parameter during the inhalation.

The determination of the indication of the amount of the ingredient delivered from the aerosol-generating material 38 may also be based on other factors, such as a concentration of the ingredient in the aerosol-generating material 38. It will be appreciated that, for a given amount of aerosol generated by the aerosol generator 36 from the aerosol-generating material 38, the amount of the ingredient in the resulting aerosol will vary depending on the concentration, in other words the amount, of the ingredient in the aerosol-generating material 38. As described above, the ingredient may be an active substance, such as nicotine, caffeine, taurine, theine, vitamins such as B6 or B12 or C, melatonin, cannabinoids, or constituents, derivatives, or combinations thereof. The ingredient may be a flavourant, an aerosol-former material or a functional material such as a pH regulator, colouring agent, preservative, binder, filler, stabilizer or antioxidant. Accordingly, the concentration of the ingredient in the aerosol-generating material 38 can be considered when determining the indication of the amount of the ingredient delivered from the aerosol-generating material 38 in order to improve the accuracy of the determination. The concentration of the ingredient in the aerosol-generating material 38 may be provided to the control circuitry by the user, for example by inputting the concentration on a user input device associated with the aerosol provision system, or control circuitry 28 may be configured to determine the concentration of the ingredient in the aerosol-generating material 38, for example in response to the consumable 30 being attached to the aerosol provision system 10. The consumable 30 may comprise an electronic chip or tag, such as an RFIG tag, which the control circuitry 28 as able to read in order to determine the concentration of the ingredient in the aerosol-generating material 38, as well as other properties of the consumable 30, such as the identify of the manufacturer or the consumable, one or more flavourants or other ingredients contained within the aerosol-generating material 38 and the volume or mass of the aerosol-generating material 38 in the consumable.

As described above, the control circuitry 28 is configured to determine an indication of the amount of the ingredient delivered. The indication may represent the actual amount of the ingredient delivered, for example a mass or volume of the ingredient delivered from the aerosol-generating material 38 to the user during the inhalation. For example, control circuitry 28 may be configured to use an algorithm or look-up table to determine the amount of the ingredient delivered during the inhalation based on the inhalation detection signals. The algorithm or look-up table may be based on empirical data related to the aerosol provision system 10, such as the maximum or average mass flow of air through the air channel 23, or an amount of the ingredient delivered for a standard inhalation profile, such as 55ml of air in a 3 second inhalation every thirty seconds (referred to as an 55/3/30 profile). If the amount of an ingredient delivered for a standard inhalation profile is known, then this can be scaled using a look-up table or an algorithm in order to determine the amount of the ingredient that is delivered for an inhalation with a different profile, such as the duration of the inhalation, the volume of aerosol delivered, the amount of electrical power delivered to the aerosol generator 36 and/or a temperature of the aerosol generator 36.

Alternatively, the indication of the amount of the ingredient delivered during the inhalation may relate to the amount of the ingredient delivered compared to a capacity of the aerosol-generating material storage area, such that the indication of the amount of the ingredient delivered indicates the amount of the ingredient and/or the aerosol-generating material remaining in the aerosol-generating material storage area. For example, the indication could be a percentage of the total amount of the aerosol-generating material present in the aerosol-generating material storage area when the aerosol-generating material storage area is full.

In some embodiments, the indication of the amount of the ingredient delivered during the inhalation is a rating on a fixed scale, for example an integer or real number between 0 and 10, where 0 is the lowest value and 10 is the highest value, although different forms and granulations of scales can also be used. In this case, an indication of 2 represents that a small amount of ingredient was delivered during the inhalation, whilst an indication of 10 represents that a maximum amount of the ingredient was delivered. This rating may be calculated by multiplying a duration of the inhalation by an indication of the operational parameter during the inhalation and applying one or more scaling factors, or by any other suitable calculation technique. Using such a rating on a scale allows for comparison between indications from different inhalations without requirement as exact or detailed a calculation as when the indication corresponds to the actual amount of the ingredient delivered.

The determination of the indication of the amount of the ingredient delivered from the aerosol-generating material 38 to the user may occur during the inhalation itself. In other words, the control circuitry 28 is configured to determine the indication of the amount of the ingredient that has been delivered as the inhalation takes place, such that the determination is ongoing during the inhalation. The determination of the indication of the amount of the ingredient delivered therefore occurs as inhalation detection signals are received from the sensor 25. For example, as described above, the control circuitry 28 may be configured to start the determination of the indication of the amount of the ingredient delivered in response to receiving inhalation detection signals from the sensor 25, or in response to a change in the inhalation detection signals received from the sensor 25. The determination of the indication of the amount of the ingredient delivered would then continue until the inhalation detection signals were no longer received from the sensor 25, or the inhalation detection signals received from the sensor 25 changed for a second time. Alternatively, the determination of the indication of the amount of the ingredient delivered could occur after the inhalation has concluded.

The indication of the amount of the ingredient delivered during an inhalation can be used to determine an indication of the amount of the ingredient delivered from the aerosol-generating material 38 to the user during each inhalation in a predetermined time period, such as a minute, an hour, a day, a week or longer. For example, the control circuitry 28 can be configured to determine an indication of the amount of the ingredient delivered during each inhalation in a rolling time period, which can then be summed to determine an indication of the total amount of the ingredient delivered from the aerosol-generating material 38 to the user in the rolling time period. As will be appreciated, a rolling time period is intended to mean the time immediately prior to any point in time, such that, for example, a rolling 24 hour period represents the 24 hour time period immediately prior to any point in time (in other words the most recent 24 hours in time at any point in time). Equally, the determination may be performed over a rolling time period of an hour, a day (24 hours), a week (7 days) or other period of time.

Additionally, the determination of the indication of the amount of the ingredient delivered from the aerosol-generating material 38 to the user during the predetermined time period may also be based on the time between each inhalation during the predetermined time period. For some ingredients, the amount of residual ingredient in the user's body system will decrease over time as the ingredient is absorbed, broken down, expelled or otherwise depleted from the user's body system. By considering the time between each inhalation and the duration of each inhalation in a predetermined time period, the indication of an amount of the ingredient delivered from the aerosol-generating material 38 to the user during the predetermined time period can indicate the amount of residual ingredient in the user's body system rather than the amount of the ingredient delivered to the user in the predetermined time period.

As will be appreciated, many users of aerosol provision systems 10 do not take single inhalations on the aerosol provision system 10, but rather perform a session on the aerosol provision system 10, where a session is a plurality of inhalations within a time period such as 1 to 2 minutes, sometimes longer such as 5 or 10 minutes. The control circuitry 28 can therefore be configured to determine an indication of the amount of the ingredient delivered from the aerosol-generating material 38 to the user over a plurality of inhalations, where a time between each of the plurality of inhalations is less than a predetermined time. The predetermined time may be set and altered by the user or the control circuitry 28, or may be a fixed value, for example based on empirical data. The predetermined time could be less than 1 minute, 1, 2, 5, 10 minutes or longer. The predetermined time can be defined as a rolling time period as described above such that each inhalation within the most recent predetermined time period is considered by the control circuitry to be part of the session. Alternatively, the predetermined time may be set such that the time between each inhalation must be less than the predetermined time in order for the inhalation to be considered by the control circuitry 28 as part of the same session. In this case, a session timer could be implemented to determine if an inhalation is part of a session, where the session timer is started when the inhalation detection signals indicate that an inhalation has been detected by the sensor, and the session timer is stopped when the duration between an inhalation and the next inhalation exceeds the predetermined time. Alternatively, as described above, a time stamp associated with each inhalation detection signal could be used to determine an indication of the amount of the ingredient delivered from the aerosol-generating material 38 to the user over the session. The determining of the indication of the amount of the ingredient delivered from the aerosol-generating material 38 to the user during the session may also be based on inhalation detection signals received for each inhalation during the session and the time in between each inhalation during the session. As described above, this determination may be performed for each inhalation in the session, for example a separate determination performed during each inhalation or after each inhalation has concluded in the session. Alternatively, the determination may be performed once, either during the session or after the entire session has concluded.

The control circuitry 28 is configured to compare the indication of the amount of the ingredient delivered to a threshold. The threshold may be set based on an amount of the ingredient delivered in a single puff (a puff threshold), for example to correspond to a safe usage limit of the ingredient and/or aerosol-generating material 38, or a safe usage limit of the aerosol provision system 10 for an inhalation, for example to prevent overheating or drying out of one or more of the components of the aerosol provision system 10. Alternatively, the threshold may correspond to a session threshold or a daily threshold, which may correspond to a safe usage limit of the ingredient and/or aerosol-generating material, or a safe usage limit of the aerosol provision system for a session and a 24 hour period respectively. While the above refers to a "safe" usage limit, it should be appreciated that this may not necessarily refer to a medically approved maximum dosage of the ingredient and/or aerosol generating material 38, but rather may refer to a recommended dosage somewhat below the medically approved maximum dosage, for example. Additionally, the threshold may be adjustable and/or set based by the user (either directly or automatically). Thus, more generally, the threshold represents an amount of the ingredient and/or aerosol generating material 38 over a given time period (e.g., an inhalation, 1 minute, 1 hour, etc.) which is typically below a medically approved maximum amount for the given time period.

As described above, the determination of the indication of an amount of an ingredient delivered may occur during the inhalation. In this case, the comparison between the indication of an amount of an ingredient delivered and the threshold may be performed during the inhalation, either continuously or periodically (such as every second or every 5 seconds). In other words, the indication of an amount of an ingredient delivered is constantly determined during the inhalation, and the value for the indication of an amount of an ingredient delivered at the current moment in time is compared to the threshold. Alternatively, comparing the indication of the amount of the ingredient delivered to the threshold can performed after each inhalation. In this case, the determination of indication of an amount of an ingredient delivered may occur either during the inhalation or after the inhalation.

The control circuitry 28 is configured provide a notification to the user based on the comparison between the indication of the amount of the ingredient delivered to the threshold. For example, a notification may be provided on the aerosol provision system 10, such as by activating an indicator light, emitting a sound from a speaker or displaying a message on a display screen on the aerosol provision device 20 and/or the consumable 30. The notification may also be a haptic notification on the aerosol provision system 10, such as a vibration or force feedback. For example, a vibration may be generated by an eccentric rotating mass (ERM) or piezoelectric actuator within the aerosol provision device 20 and/or the consumable 30, or a force may be generated by a motor within the aerosol provision device 20 and/or the consumable 30. The notification could also be a change in a mode of operation of the aerosol provision system 10 which the user would detect, such as switching off, disabling or otherwise preventing electrical power from being supplied to the aerosol generator 36. For example, the aerosol generator 36 could be disabled for a period of time, such as 5 seconds, 10 seconds, a minute or longer.

Alternatively, or in addition, the notification may be provided on an application on a remote device. For example, the user of the aerosol provision system 10 may have a remote device associated with, but separate from, the aerosol provision system 10, and the control circuitry 28 is configured to communicate with the remote device, for example by Bluetooth, Bluetooth Low Energy (BLE), ANT+, Wi-Fi or any other suitable wireless communication method. The control circuitry 28 can be configured to communicate with the remote device such that the notification is provided to the user on the remote device, such as on an application installed on the remote device. For example, a message may be displayed on a display screen on the remote device, an indicator light activated, a sound emitted from a speaker or a haptic notification means on the remote device as described above. The remote device may include any suitable electronic device that can be communicatively coupled to the aerosol provision system 10. For example, the remote device may include a mobile device (such as a smartphone), a PDA, a personal computer, laptop, tablet, smartwatch, etc.

Further, one or more parameters associated with the notification may be adjustable by the user. For example, the user may be able to adjust the number, brightness and/or colour of the indictor light that is activated, the volume, pitch and or duration of the sound emitted and/or the message that is displayed. The user may also be able to adjust one or more parameters of the haptic notification. For example the user may be able to adjust the duration, magnitude and/or pattern of the vibrations or forces provided by the actuator and motor respectively.

The notification may be configurable by the user. In other words, the user may be able to adjust the one or more parameters associated with the notification on the aerosol provision system 10 and/or the remote device regardless of whether the notification is provided on the aerosol provision system 10 or the remote device. For example, the user may be able to use the application on the remote device to adjust one or more of the parameters associated with the notification even though the notification itself is provided on the aerosol provision system 10. For example, the user may disable notifications during an inhalation such that notifications are only received when an inhalation is not detected by the sensor 25.

In some embodiments, the control circuitry 28 is configured to provide the notification to the user when the indication of an amount of an ingredient delivered from the aerosol-generating material to the user during the inhalation exceeds the threshold. The indication of the amount of the ingredient delivered from the aerosol-generating material to the user during the inhalation might represent a proportion or percentage of the amount of the ingredient delivered from the aerosol-generating material to the user during the inhalation compared to the threshold. For example, the indication might be a percentage of the threshold, such as 10%, 20%, 50%, 80% or 110%, and the notification is provided to the user when the percentage is greater that 100%. In the embodiments where the comparison between the indication of the amount of the ingredient delivered and the threshold occurs during the inhalation, the notification can be provided to the user during the inhalation as soon as the amount of the ingredient delivered from the aerosol-generating material to the user exceeds the puff threshold.

Alternatively, the notification could be provided to the user when the indication of the amount of the ingredient delivered is within a range of the threshold, such as 50%, 75% or 90% of the threshold. This ensures that the user provided with the notification before the threshold is exceeded. In this case, the notification could indicate to the user how close to the threshold the indication of an amount of an ingredient delivered is, for example by displaying the ratio of the values or by a visual means such as a progress bar or by lighting a particular number of indicator lights on the aerosol provision system or the remote device.

In some embodiments, the aerosol provision system 10 is configured to display, or otherwise communicate, the indication of the amount of the ingredient delivered to the user. For instance, the aforementioned progress bar or the like could be displayed to the user. This may be displayed at all times, or in response to a user request for this information. However, when the indication of the amount of the ingredient delivered is within a range of the threshold, or exceeds the threshold, the control circuitry 28 provides the notification to the user. For instance, when the progress bar fills to 90%, with 100% representing the threshold, the control circuitry 28 may provide the notification to the user, which may for example, be a change in the display (e.g., the colour of the progress bar may change) or a haptic or audible notification may be provided.

In some embodiments, the control circuitry 28 is configured to determine default user behaviour based on the indication of the amount of the ingredient delivered from the aerosol-generating material 38 to the user. In other words, the control circuitry 28 is configured to detect patterns in one or more inhalations by the user based on the indication of the amount of the ingredient delivered from the aerosol-generating material 38 to the user. The default user behaviour can also be determined from the data determined by the control circuitry 28 for one or more inhalations which is used to generate the indication of the amount of the ingredient delivered, such as the duration of an inhalation, the duration of a session, a time between inhalations, the amount of electrical power delivered to the aerosol generator 36 during the inhalation, a power level or setting for the aerosol generator 36 for the inhalation and the type and/or concentration of one or more of the ingredients in the aerosol-generating material 38. These patterns are then used to default user behaviour with respect to the amount of the ingredient delivered from the aerosol-generating material 38 to the user during an inhalation.

The default user behaviour can be determined over an inhalation, such that the default user behaviour relates to a typical inhalation performed by the user on the aerosol provision system 10. For example, when the user first uses the aerosol provision system 10, in other words where only a single inhalation has been detected, the default user behaviour may correspond to the indication of the amount of the ingredient delivered for that inhalation. As the user performs more inhalations on the aerosol provision system 10, the default user behaviour can be updated based on the indication of the amount of the ingredient delivered for each subsequent inhalation, thereby refining the typical inhalation performed by the user on the aerosol provision system 10.

The indication of the of the amount of the ingredient delivered for each inhalation can also be used to determine default user behaviour over a session; in other words, the default user behaviour is determined over a plurality of inhalations, where a time between each of the plurality of inhalations is less than a predetermined time as described above. The indication of the amount of the ingredient delivered for each inhalation can also be used to determine default user behaviour over another period, such as a rolling time period or a rolling 24 hour period as described above, a week, a month and/or a year.

Additionally, the control circuitry 28 can continually update the determined default user behaviour based on changes in the indication of the amount of the ingredient delivered from the aerosol-generating material 38 to the user for inhalations over time. For example, where the control circuitry 28 is configured to determine a time between an inhalation and the next inhalation based on the inhalation detection signals, the default user behaviour can also be determined based on the time between the inhalation and the next inhalation. This allows patterns of behaviour to be detected for the user, such as if the user takes a series of puffs, such as a session described above, then has an extended period between sessions, such as 30 minutes, 1 hour or longer, or whether the user takes a small number of inhalations, such as 1 or 2, but spaced more regularly, such as every 10 of 20 minutes. Equally, the data collected may allows the control circuitry 28 to determine particular times of the day when the user takes more inhalations, such as in the mornings or the evenings, or if the number and duration of inhalations in a session change during a day. For example, the user may have a session comprising a plurality of long inhalations with a high power setting in the morning, but sessions in the evening comprise fewer, short inhalations with a lower power setting. The user may perform more inhalations on weekdays, whilst over the course of a month or a year the data may indicate that the user is performing fewer inhalations, for example due to the user trying to cut down their usage of the aerosol provision system. Such default behaviour can be determined based on the indication of the amount of the ingredient delivered from the aerosol-generating material 38 to the user for a plurality of inhalations.

As described above, for some ingredients the amount of residual ingredient in the user's body system will decrease over time as the ingredient is absorbed, broken down, expelled or otherwise depleted from the user's body system. The indication of an amount of the ingredient delivered from the aerosol-generating material 38 to the user can indicate the amount of residual ingredient in the user's body system rather than the amount of the ingredient delivered to the user in the predetermined time period, and therefore the default user behaviour can also be based on the amount of residual ingredient in the user's body system.

According to an exemplary embodiment of the invention, the control circuitry 28 is configured to determine or alter the threshold described above based on the default user behaviour, thereby personalising or otherwise tailoring the operation of the aerosol provision system 10 to the user. Alternatively or in addition, the user may be able to alter one or more of the thresholds, for example by providing an input on an input device on the aerosol provision device 20 or consumable 30, or through an application on an associated remote device, thereby giving the user additional control over the operation of the aerosol provision system 10.

The control circuitry 28 may also be configured to alter a mode of operation of the aerosol generation system 10 based on the default user behaviour, such as an amount of electrical power supplied to the aerosol generator 36 by the power source 14, the temperature of the aerosol generator 36, a sensitivity or detection threshold on the sensor 25, the colour and/or number of light indicators illuminated and/or the volume, pitch and/or duration of a sound emitted on the aerosol provision device 20 for an inhalation.

For example, if it is determined that the user takes longs inhalations, such as greater than 10 seconds, the control circuitry 28 can be configured to alter the amount of electrical power supplied to the aerosol generator 36 by the power source 14 during the inhalation in order to prevent dry out or overheating of the aerosol generator 36. The power supplied to the aerosol generator 36 may be set to an initial value or power setting, and then reduced as the inhalation continues. Alternatively, if is determined that the user takes very small or gentle inhalations, for example with a low air speed or mass flow, the control circuitry 28 can be configured to change a sensitivity or detection threshold on the sensor 25 to ensure that an inhalation is properly detected for the user.

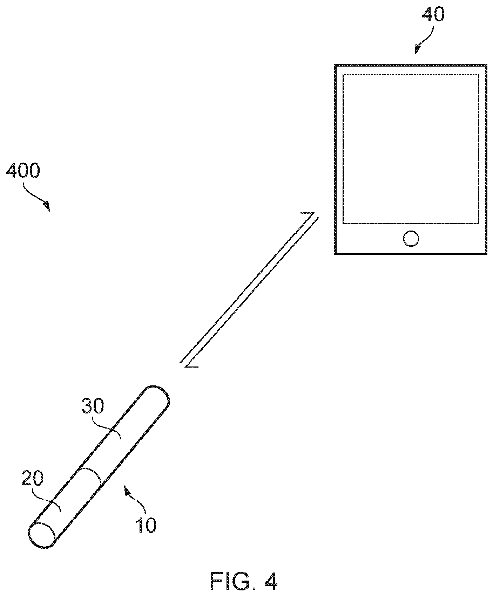

Figure 4 illustrates a system 400 comprising an aerosol provision system 10 configured to generate aerosol from an aerosol-generating material 38, such as described above. The system 400 also comprises a computer 40 configured to receive inhalation detection signals from a sensor 25 configured to determine an indication of an amount of an ingredient delivered from the aerosol-generating material 38 aerosolized by an aerosol generator 36 to the user during the inhalation based on inhalation detection signals received from a sensor 25 of the aerosol provision system 10. The computer 40 is also configured to compare the indication of the amount of the ingredient to a threshold and provide a notification to the user based on the comparison between the indication of the amount of the ingredient delivered to the threshold.

As described above and illustrated in Figure 4, the computer 40 may be a remote device associated with the user and in communication with the aerosol provision system 10. Accordingly, it will be appreciated that the functions of the control circuitry described herein, such determining an indication of an amount of an ingredient delivered, determining default user behaviour, comparing the indication of the amount of the ingredient to a threshold and providing a notification to the user may be performed by a computer 40 separate from the aerosol provision system 10, such as a remote device.

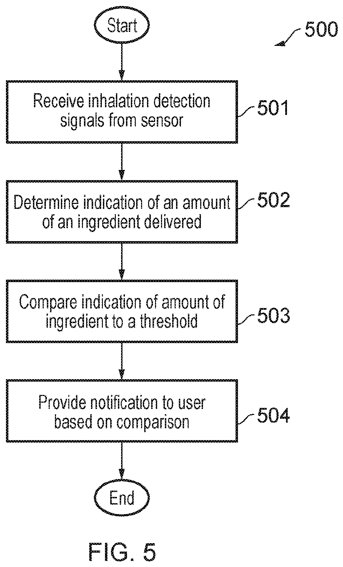

Figure 5 is a flow chart of a method 500 of determining an amount of an ingredient delivered to a user of an aerosol provision system 10. The method begins at step 501, where inhalation detection signals are received from a sensor configured to detect an inhalation on the aerosol provision system by a user of aerosol provision system. At step 502, an indication of an amount of an ingredient delivered from an aerosol-generating material aerosolized by an aerosol generator to the user during the inhalation is determined based on the inhalation detection signals. At step 503 the indication of the amount of the ingredient is compared to a threshold. At step 504 a notification is provided to the user based on the comparison between the indication of the amount of the ingredient delivered to the threshold.

The method 500 illustrated in Figure 5 may be stored as instructions on a computer readable storage medium, such that when the instructions are executed by a processor, the method 500 described above is performed. The computer readable storage medium may be non-transitory.

Although it has been described above that the indication of the amount of the ingredient delivered is compared to a threshold, and subsequently a notification to the user is provided based on the comparison between the indication of the amount of the ingredient delivered to the threshold, it should be appreciated that in some implementations a plurality of thresholds (e.g. two) may be provided, and the control circuitry 28 is configured to compare the indication of the amount of the ingredient delivered to each of the thresholds. For example, if the amount of ingredient delivered exceeds a first amount, a notification may be provided alerting the user that a first level of ingredient has been delivered. Continued use may cause the amount of ingredient delivered to exceed a second amount, at which point a subsequent notification may be provided alerting the user that a second level of ingredient has been delivered. Alternatively, the first and second thresholds may define a range for the amount of ingredient delivered between which no notification is issued. If the amount of ingredient delivered is above or below the range, then the control circuity 28 may provide a notification accordingly. In the context of the lower threshold, this may be set by a user in accordance with a nicotine cessation program or the like, and as such the notification may provide the user with a message stating the amount of ingredient delivered is below the threshold. The notification and/or action taken by the control circuitry 28 may be the same or different for the comparison of the ingredient delivered compared to the different thresholds.

As described above, the present disclosure relates to (but it not limited to) an aerosol provision system comprising an aerosol generator configured to aerosolize an aerosol-generating material. The aerosol provision system also comprises a sensor configured to detect an inhalation on the aerosol provision system by a user of the aerosol provision system, and output corresponding inhalation detection signals to control circuitry. The control circuitry is configured to determine an indication of an amount of an ingredient delivered from the aerosol generating material to the user during the inhalation based on the inhalation detection signals, compare the indication of the amount of the ingredient delivered to a threshold, and provide a notification to the user based on the comparison between the indication of the amount of the ingredient delivered to the threshold.

Thus, there has been described an aerosol provision system, a system comprising an aerosol provision system and a computer, a method of operating an aerosol provision system, and a computer readable storage medium.

The various embodiments described herein are presented only to assist in understanding and teaching the claimed features. These embodiments are provided as a representative sample of embodiments only, and are not exhaustive and/or exclusive. It is to be understood that advantages, embodiments, examples, functions, features, structures, and/or other aspects described herein are not to be considered limitations on the scope of the invention as defined by the claims, and that other embodiments may be utilised and modifications may be made without departing from the scope of the claimed invention. Various embodiments of the invention may suitably comprise, consist of, or consist essentially of, appropriate combinations of the disclosed elements, components, features, parts, steps, means, etc., other than those specifically described herein.

## Claims

1. An aerosol provision system (10) comprising:
an aerosol generator (36) configured to aerosolize an aerosol-generating material (38); and
a sensor (25) configured to detect an inhalation on the aerosol provision system by a user of the aerosol provision system, and output corresponding inhalation detection signals to a control circuitry (28) of the aerosol provision system (10);
wherein the control circuitry (28) is configured to:
determine an indication of an amount of an ingredient delivered from the aerosol-generating material to the user during the inhalation based on the inhalation detection signals;
compare the indication of the amount of the ingredient delivered to a threshold; and
provide a notification to the user based on the comparison between the indication of the amount of the ingredient delivered to the threshold;
wherein the control circuitry is configured to determine default user behaviour based on the indication of the amount of the ingredient delivered from the aerosol-generating material to the user,
wherein the default user behaviour is determined over an inhalation; or
wherein the control circuitry is configured to determine a time between the inhalation and a next inhalation based on the inhalation detection signals, and wherein determining default user behaviour is also based on the time between the inhalation and the next inhalation; or
wherein the default user behaviour is determined over a plurality of inhalations, and a time between each of the plurality of inhalations is less than a predetermined time; or
wherein the default user behaviour is determined over a rolling time period;
and **characterised in that** the control circuitry (28) is configured to determine the threshold based on the determined default user behaviour.

2. The aerosol provision system of claim 1, wherein comparing the indication of the amount of the ingredient delivered to the threshold is performed based on an indication of the amount of the ingredient delivered from the aerosol-generating material to the user during each inhalation in a predetermined period, wherein optionally, the predetermined period is an hour, 24 hours or a week.

3. The aerosol provision system of any one of claims 1 to 2, wherein comparing the indication of the amount of the ingredient delivered to the threshold is performed after each inhalation, or wherein comparing the indication of the amount of the ingredient delivered to the threshold is performed during the inhalation, wherein optionally, comparing the indication of the amount of the ingredient delivered to the threshold is performed at a predetermined interval during the inhalation.

4. The aerosol provision system of any one of claims 1 to 3, wherein the determination of the indication of the amount of the ingredient delivered from the aerosol-generating material to the user is further based on a concentration of the ingredient in the aerosol-generating material.

5. The aerosol provision system of any one of claims 1 to 4, wherein the ingredient is nicotine, caffeine, taurine, theine, a vitamin, melatonin, or a cannabinoid.

6. The aerosol provision system of any one of claims 1 to 5, wherein the notification is provided to the user when the indication of the amount of the ingredient delivered exceeds the threshold.

7. The aerosol provision system of any one of claims 1 to 6, wherein the notification is provided on the aerosol provision system, or on an application on a remote device.

8. The aerosol provision system of any one of claims 1 to 7, wherein the notification is a haptic notification, wherein optionally a parameter of the haptic notification is adjustable by the user of the aerosol provision system.

9. The aerosol provision system of any one of claims 1 to 8, wherein the notification is configurable by the user, and / or wherein the notification is provided to the user based on the comparison between the indication of the amount of the ingredient delivered to a plurality of thresholds.

10. A system (400) comprising:
an aerosol provision system (10) configured to generate aerosol from an aerosol-generating material (38); and
a computer (40) configured to:
determine an indication of an amount of an ingredient delivered from the aerosol-generating material aerosolized by an aerosol generator (36) to the user during the inhalation based on inhalation detection signals received from a sensor (25) of the aerosol provision system;
compare the indication of the amount of the ingredient to a threshold; and
provide a notification to the user based on the comparison between the indication of the amount of the ingredient delivered to the threshold,
wherein the computer is configured to determine default user behaviour based on the indication of the amount of the ingredient delivered from the aerosol-generating material to the user,
wherein the default user behaviour is determined over an inhalation;
or
wherein the control circuitry is configured to determine a time between the inhalation and a next inhalation based on the inhalation detection signals, and wherein determining default user behaviour is also based on the time between the inhalation and the next inhalation; or
wherein the default user behaviour is determined over a plurality of inhalations, and a time between each of the plurality of inhalations is less than a predetermined time; or
wherein the default user behaviour is determined over a rolling time period;
and **characterised in that** the computer (40) is configured to determine the threshold based on the determined default user behaviour.

11. A method of operating an aerosol provision system (10) comprising:
receiving inhalation detection signals from a sensor (25) configured to detect an inhalation on the aerosol provision system by a user of aerosol provision system;
determining an indication of an amount of an ingredient delivered from an aerosol-generating material (38) aerosolized by an aerosol generator (36) to the user during the inhalation based on the inhalation detection signals;
comparing the indication of the amount of the ingredient to a threshold; and
providing a notification to the user based on the comparison between the indication of the amount of the ingredient delivered to the threshold,
wherein the method further comprises determining default user behaviour based on the indication of the amount of the ingredient delivered from the aerosol-generating material to the user;
wherein the default user behaviour is determined over an inhalation; or
wherein the control circuitry is configured to determine a time between the inhalation and a next inhalation based on the inhalation detection signals, and wherein determining default user behaviour is also based on the time between the inhalation and the next inhalation; or
wherein the default user behaviour is determined over a plurality of inhalations, and a time between each of the plurality of inhalations is less than a predetermined time; or
wherein the default user behaviour is determined over a rolling time period;
and **characterised in that** the threshold is determined based on the determined default user behaviour.

12. A computer readable storage medium comprising instructions which, when executed by a processor, performs a method comprising:
receiving inhalation detection signals from a sensor (25) configured to detect an inhalation on the aerosol provision system (10) by a user of aerosol provision system;
determining an indication of an amount of an ingredient delivered from an aerosol-generating material (38) aerosolized by an aerosol generator (36) to the user during the inhalation based on the inhalation detection signals;
comparing the indication of the amount of the ingredient to a threshold; and
providing a notification to the user based on the comparison between the indication of the amount of the ingredient delivered to the threshold,
wherein the method further comprises determining default user behaviour based on the indication of the amount of the ingredient delivered from the aerosol-generating material to the user;
wherein the default user behaviour is determined over an inhalation; or
wherein the control circuitry is configured to determine a time between the inhalation and a next inhalation based on the inhalation detection signals, and wherein determining default user behaviour is also based on the time between the inhalation and the next inhalation; or
wherein the default user behaviour is determined over a plurality of inhalations, and a time between each of the plurality of inhalations is less than a predetermined time; or
wherein the default user behaviour is determined over a rolling time period;
and **characterised in that** the threshold is determined based on the determined default user behaviour.

## Patentansprüche

1. Aerosolbereitstellungssystem (10), umfassend:
einen Aerosolgenerator (36), der konfiguriert ist zum Aerosolisieren eines Aerosol erzeugenden Materials (38); und
einen Sensor (25), der konfiguriert ist zum Erkennen einer Inhalation am Aerosolbereitstellungssystem durch einen Benutzer des Aerosolbereitstellungssystems und Ausgeben entsprechender Inhalationserkennungssignale an eine Steuerschaltung (28) des Aerosolbereitstellungssystems (10);
wobei die Steuerschaltung (28) konfiguriert ist zum:
Bestimmen einer Angabe einer Menge eines Inhaltsstoffs, der während der Inhalation aus dem Aerosol erzeugenden Material an den Benutzer abgegeben wird, basierend auf den Inhalationserkennungssignalen;
Vergleichen der Angabe der Menge des abgegebenen Inhaltsstoffs mit einem Schwellenwert; und
Bereitstellen einer Benachrichtigung an den Benutzer basierend auf dem Vergleich zwischen der Angabe der Menge des abgegebenen Inhaltsstoffs mit dem Schwellenwert;
wobei die Steuerschaltung konfiguriert ist zum Bestimmen eines Standardbenutzerverhaltens basierend auf der Angabe der Menge des Inhaltsstoffs, die von dem Aerosol erzeugenden Material an den Benutzer abgegeben wird,
wobei das Standardbenutzerverhalten über eine Inhalation bestimmt wird; oder
wobei die Steuerschaltung konfiguriert ist zum Bestimmen einer Zeit zwischen der Inhalation und einer nächsten Inhalation basierend auf den Inhalationserkennungssignalen, und wobei das Bestimmen eines Standardbenutzerverhaltens auch auf der Zeit zwischen der Inhalation und der nächsten Inhalation basiert; oder
wobei das Standardbenutzerverhalten über eine Vielzahl von Inhalationen bestimmt wird, und eine Zeit zwischen jeder der Vielzahl von Inhalationen kürzer ist als eine vorgegebene Zeit; oder
wobei das Standardbenutzerverhalten über einen gleitenden Zeitraum bestimmt wird;
und **dadurch gekennzeichnet, dass** die Steuerschaltung (28) konfiguriert ist zum Bestimmen des Schwellenwerts basierend auf dem bestimmten Standardbenutzerverhalten.

2. Aerosolbereitstellungssystem nach Anspruch 1, wobei das Vergleichen der Angabe der Menge des abgegebenen Inhaltsstoffs mit dem Schwellenwert basierend auf einer Angabe der Menge des Inhaltsstoffs erfolgt, die von dem Aerosol erzeugenden Material an den Benutzer während jeder Inhalation in einem vorgegebenen Zeitraum abgegeben wird, wobei der vorgegebene Zeitraum optional eine Stunde, 24 Stunden oder eine Woche beträgt.

3. Aerosolbereitstellungssystem nach einem der Ansprüche 1 bis 2, wobei das Vergleichen der Angabe der Menge des abgegebenen Inhaltsstoffs mit dem Schwellenwert nach jeder Inhalation erfolgt, oder wobei das Vergleichen der Angabe der Menge des abgegebenen Inhaltsstoffs mit dem Schwellenwert während der Inhalation erfolgt, wobei optional, das Vergleichen der Angabe der Menge des abgegebenen Inhaltsstoffs mit dem Schwellenwert in einem vorgegebenen Intervall während der Inhalation erfolgt.

4. Aerosolbereitstellungssystem nach einem der Ansprüche 1 bis 3, wobei das Bestimmen der Angabe der Menge des Inhaltsstoffs, die von dem Aerosol erzeugenden Material an den Benutzer abgegeben wird, weiter auf einer Konzentration des Inhaltsstoffs in dem Aerosol erzeugenden Material basiert.

5. Aerosolbereitstellungssystem nach einem der Ansprüche 1 bis 4, wobei der Inhaltsstoff Nikotin, Koffein, Taurin, Thein, ein Vitamin, Melatonin oder ein Cannabinoid ist.

6. Aerosolbereitstellungssystem nach einem der Ansprüche 1 bis 5, wobei die Benachrichtigung dem Benutzer bereitgestellt wird, wenn die Angabe der Menge des abgegebenen Inhaltsstoffs den Schwellenwert überschreitet.

7. Aerosolbereitstellungssystem nach einem der Ansprüche 1 bis 6, wobei die Benachrichtigung über das Aerosolbereitstellungssystem oder über eine Anwendung auf einer entfernten Vorrichtung bereitgestellt wird.

8. Aerosolbereitstellungssystem nach einem der Ansprüche 1 bis 7, wobei die Benachrichtigung eine haptische Benachrichtigung ist, wobei optional ein Parameter der haptischen Benachrichtigung durch den Benutzer des Aerosolbereitstellungssystems einstellbar ist.

9. Aerosolbereitstellungssystem nach einem der Ansprüche 1 bis 8, wobei die Benachrichtigung durch den Benutzer konfigurierbar ist, und / oder wobei die Benachrichtigung dem Benutzer basierend auf dem Vergleich zwischen der Angabe der Menge des abgegebenen Inhaltsstoffs und einer Vielzahl von Schwellenwerten bereitgestellt wird.

10. System (400), umfassend:
ein Aerosolbereitstellungssystem (10), das konfiguriert ist zum Erzeugen von Aerosol aus einem Aerosol erzeugenden Material (38); und
einen Computer (40), der konfiguriert ist zum:
Bestimmen einer Angabe einer Menge eines Inhaltsstoffs, die von dem Aerosol erzeugenden Material, das von einem Aerosolgenerator (36) aerosoliert wird, während der Inhalation an den Benutzer abgegeben wird, basierend auf Inhalationserkennungssignalen, die von einem Sensor (25) des Aerosolbereitstellungssystems empfangen werden;
Vergleichen der Angabe der Menge des Inhaltsstoffs mit einem Schwellenwert; und
Bereitstellen einer Benachrichtigung an den Benutzer basierend auf dem Vergleich zwischen der Angabe der Menge des abgegebenen Inhaltsstoffs mit dem Schwellenwert,
wobei der Computer konfiguriert ist zum Bestimmen eines Standardbenutzerverhaltens basierend auf der Angabe der Menge des Inhaltsstoffs, die von dem Aerosol erzeugenden Material an den Benutzer abgegeben wird,
wobei das Standardbenutzerverhalten über eine Inhalation bestimmt wird; oder
wobei die Steuerschaltung konfiguriert ist zum Bestimmen einer Zeit zwischen der Inhalation und einer nächsten Inhalation basierend auf den Inhalationserkennungssignalen, und wobei das Bestimmen eines Standardbenutzerverhaltens auch auf der Zeit zwischen der Inhalation und der nächsten Inhalation basiert; oder
wobei das Standardbenutzerverhalten über eine Vielzahl von Inhalationen bestimmt wird, und eine Zeit zwischen jeder der Vielzahl von Inhalationen kürzer ist als eine vorgegebene Zeit; oder
wobei das Standardbenutzerverhalten über einen gleitenden Zeitraum bestimmt wird;
und **dadurch gekennzeichnet, dass** der Computer (40) konfiguriert ist zum Bestimmen des Schwellenwerts basierend auf dem bestimmten Standardbenutzerverhalten.

11. Verfahren zum Betreiben eines Aerosolbereitstellungssystems (10), umfassend:
Empfangen von Inhalationserkennungssignalen von einem Sensor (25), der konfiguriert ist zum Erkennen einer Inhalation am Aerosolbereitstellungssystem durch einen Benutzer eines Aerosolbereitstellungssystems;
Bestimmen einer Angabe einer Menge eines Inhaltsstoffs, die aus einem Aerosol erzeugenden Material (38), das von einem Aerosolgenerator (36) aerosoliert wird, während der Inhalation an den Benutzer abgegeben wird, basierend auf den Inhalationserkennungssignalen;
Vergleichen der Angabe der Menge des Inhaltsstoffs mit einem Schwellenwert;
und
Bereitstellen einer Benachrichtigung an den Benutzer basierend auf dem Vergleich zwischen der Angabe der Menge des abgegebenen Inhaltsstoffs und dem Schwellenwert,
wobei das Verfahren weiter ein Bestimmen eines Standardbenutzerverhaltens basierend auf der Angabe der Menge des Inhaltsstoffs umfasst, der von dem Aerosol erzeugenden Material an den Benutzer abgegeben wird;
wobei das Standardbenutzerverhalten über eine Inhalation bestimmt wird; oder
wobei die Steuerschaltung konfiguriert ist zum Bestimmen einer Zeit zwischen der Inhalation und einer nächsten Inhalation basierend auf den Inhalationserkennungssignalen, und wobei das Bestimmen eines Standardbenutzerverhaltens auch auf der Zeit zwischen der Inhalation und der nächsten Inhalation basiert; oder
wobei das Standardbenutzerverhalten über eine Vielzahl von Inhalationen bestimmt wird, und eine Zeit zwischen jeder der Vielzahl von Inhalationen kürzer ist als eine vorgegebene Zeit; oder
wobei das Standardbenutzerverhalten über einen gleitenden Zeitraum bestimmt wird;
und **dadurch gekennzeichnet, dass** der Schwellenwert basierend auf dem bestimmten Standardbenutzerverhalten bestimmt wird.

12. Computerlesbares Speichermedium, umfassend Anweisungen, die bei Ausführung durch einen Prozessor ein Verfahren ausführen, umfassend:
Empfangen von Inhalationserkennungssignalen von einem Sensor (25), der konfiguriert ist zum Erkennen einer Inhalation am Aerosolbereitstellungssystem (10) durch einen Benutzer eines Aerosolbereitstellungssystems;
Bestimmen einer Angabe einer Menge eines Inhaltsstoffs, die aus einem Aerosol erzeugenden Material (38), das von einem Aerosolgenerator (36) aerosoliert wird, während der Inhalation an den Benutzer abgegeben wird, basierend auf den Inhalationserkennungssignalen;
Vergleichen der Angabe der Menge des Inhaltsstoffs mit einem Schwellenwert;
und
Bereitstellen einer Benachrichtigung an den Benutzer basierend auf dem Vergleich zwischen der Angabe der Menge des abgegebenen Inhaltsstoffs und dem Schwellenwert,
wobei das Verfahren weiter ein Bestimmen eines Standardbenutzerverhaltens basierend auf der Angabe der Menge des Inhaltsstoffs umfasst, der von dem Aerosol erzeugenden Material an den Benutzer abgegeben wird;
wobei das Standardbenutzerverhalten über eine Inhalation bestimmt wird; oder
wobei die Steuerschaltung konfiguriert ist zum Bestimmen einer Zeit zwischen der Inhalation und einer nächsten Inhalation basierend auf den Inhalationserkennungssignalen, und wobei das Bestimmen eines Standardbenutzerverhaltens auch auf der Zeit zwischen der Inhalation und der nächsten Inhalation basiert; oder
wobei das Standardbenutzerverhalten über eine Vielzahl von Inhalationen bestimmt wird, und eine Zeit zwischen jeder der Vielzahl von Inhalationen kürzer ist als eine vorgegebene Zeit; oder
wobei das Standardbenutzerverhalten über einen gleitenden Zeitraum bestimmt wird;
und **dadurch gekennzeichnet, dass** der Schwellenwert basierend auf dem bestimmten Standardbenutzerverhalten bestimmt wird.

## Revendications

1. Système de fourniture d'aérosol (10) comprenant :
un générateur (36) d'aérosol configuré pour aérosoliser un matériau (38) générateur d'aérosol ; et
un capteur (25) configuré pour détecter une inhalation sur le système de fourniture d'aérosol par un utilisateur du système de fourniture d'aérosol, et émettre des signaux de détection d'inhalation correspondants à destination d'une circuiterie (28) de commande du système de fourniture d'aérosol (10) ;
dans lequel la circuiterie (28) de commande est en outre configurée pour :
déterminer une indication d'une quantité d'un ingrédient distribué à partir du matériau générateur d'aérosol à l'utilisateur pendant l'inhalation sur la base des signaux de détection d'inhalation ;
comparer l'indication de la quantité de l'ingrédient distribué à un seuil ; et fournir une notification à l'utilisateur sur la base de la comparaison entre l'indication de la quantité de l'ingrédient distribué au seuil ;
dans lequel la circuiterie de commande est configurée pour déterminer un comportement d'utilisateur par défaut sur la base de l'indication de la quantité de l'ingrédient distribué à partir du matériau générateur d'aérosol à l'utilisateur,
dans lequel le comportement d'utilisateur par défaut est déterminé sur une inhalation ; ou dans lequel la circuiterie de commande est configurée pour déterminer un temps entre l'inhalation et une inhalation suivante sur la base des signaux de détection d'inhalation, et dans lequel la détermination du comportement par défaut de l'utilisateur est également basée sur le temps entre l'inhalation et la prochaine inhalation ; ou
dans lequel le comportement d'utilisateur par défaut est déterminé sur une pluralité d'inhalations, et un temps entre chacune de la pluralité d'inhalations est inférieur à un temps prédéterminé ; ou
dans lequel le comportement d'utilisateur par défaut est déterminé sur une période de temps glissante ; et **caractérisé en ce que** la circuiterie (28) de commande est configurée pour déterminer le seuil sur la base du comportement d'utilisateur par défaut déterminé.

2. Système de fourniture d'aérosol selon la revendication 1, dans lequel la comparaison de l'indication de la quantité de l'ingrédient distribué au seuil est réalisée sur la base d'une indication de la quantité de l'ingrédient distribué à partir du matériau générateur d'aérosol à l'utilisateur pendant chaque inhalation dans une période prédéterminée, dans lequel optionnellement, la période prédéterminée est une période d'une heure, de 24 heures ou d'une semaine.

3. Système de fourniture d'aérosol selon l'une quelconque des revendications 1 à 2, dans lequel la comparaison de l'indication de la quantité de l'ingrédient distribué au seuil est réalisée après chaque inhalation, ou dans lequel la comparaison de l'indication de la quantité de l'ingrédient distribué au seuil est réalisée pendant l'inhalation, dans lequel optionnellement, la comparaison de l'indication de la quantité de l'ingrédient distribué au seuil est réalisée à un intervalle prédéterminé pendant l'inhalation.

4. Système de fourniture d'aérosol selon l'une quelconque des revendications 1 à 3, dans lequel la détermination de l'indication de la quantité de l'ingrédient distribué à partir du matériau générateur d'aérosol à l'utilisateur est en outre basée sur une concentration de l'ingrédient dans le matériau générateur d'aérosol.

5. Système de fourniture d'aérosol selon l'une quelconque des revendications 1 à 4, dans lequel l'ingrédient est de la nicotine, de la caféine, de la taurine, de la théine, une vitamine, de la mélatonine ou un cannabinoïde.

6. Système de fourniture d'aérosol selon l'une quelconque des revendications 1 à 5, dans lequel la notification est fournie à l'utilisateur lorsque l'indication de la quantité de l'ingrédient distribué dépasse le seuil.

7. Système de fourniture d'aérosol selon l'une quelconque des revendications 1 à 6, dans lequel la notification est fournie sur le système de fourniture d'aérosol, ou sur une application sur un dispositif distant.

8. Système de fourniture d'aérosol selon l'une quelconque des revendications 1 à 7, dans lequel la notification est une notification haptique, dans lequel optionnellement un paramètre de la notification haptique est réglable par l'utilisateur du système de fourniture d'aérosol.

9. Système de fourniture d'aérosol selon l'une quelconque des revendications 1 à 8, dans lequel la notification est configurable par l'utilisateur, et/ou dans lequel la notification est fournie à l'utilisateur sur la base de la comparaison entre l'indication de la quantité de l'ingrédient distribué à une pluralité de seuils.

10. Système (400) comprenant :
un système de fourniture d'aérosol (10) configuré pour générer un aérosol à partir d'un matériau (38) générateur d'aérosol ; et
un ordinateur (40) configuré pour :
déterminer une indication d'une quantité d'un ingrédient distribué à partir du matériau générateur d'aérosol aérosolisé par un générateur (36) d'aérosol à l'utilisateur pendant l'inhalation sur la base de signaux de détection d'inhalation reçus d'un capteur (25) du système de fourniture d'aérosol ;
comparer l'indication de la quantité de l'ingrédient à un seuil ; et fournir une notification à l'utilisateur sur la base de la comparaison entre l'indication de la quantité de l'ingrédient distribué au seuil,
dans lequel l'ordinateur est configuré pour déterminer un comportement d'utilisateur par défaut sur la base de l'indication de la quantité de l'ingrédient distribué à partir du matériau générateur d'aérosol à l'utilisateur,
dans lequel le comportement d'utilisateur par défaut est déterminé sur une inhalation ; ou
dans lequel la circuiterie de commande est configurée pour déterminer un temps entre l'inhalation et une inhalation suivante sur la base des signaux de détection d'inhalation, et dans lequel la détermination du comportement par défaut de l'utilisateur est également basée sur le temps entre l'inhalation et la prochaine inhalation ; ou
dans lequel le comportement d'utilisateur par défaut est déterminé sur une pluralité d'inhalations, et un temps entre chacune de la pluralité d'inhalations est inférieur à un temps prédéterminé ; ou
dans lequel le comportement d'utilisateur par défaut est déterminé sur une période de temps glissante ;
et **caractérisé en ce que** l'ordinateur (40) est configuré pour déterminer
le seuil basé sur le comportement d'utilisateur par défaut déterminé.

11. Procédé de fonctionnement d'un système de fourniture d'aérosol (10) comprenant :
la réception de signaux de détection d'inhalation en provenance d'un capteur (25) configuré pour détecter une inhalation sur le système de fourniture d'aérosol par un utilisateur du système de fourniture d'aérosol ;
la détermination d'une indication d'une quantité d'un ingrédient distribué à partir d'un matériau (38) générateur d'aérosol aérosolisé par un générateur (36) d'aérosol à l'utilisateur pendant l'inhalation sur la base des signaux de détection d'inhalation ;
la comparaison de l'indication de la quantité de l'ingrédient à un seuil ; et
la fourniture d'une notification à l'utilisateur sur la base de la comparaison entre l'indication de la quantité de l'ingrédient distribué au seuil,
dans lequel le procédé comprend en outre la détermination du comportement d'utilisateur par défaut sur la base de l'indication de la quantité de l'ingrédient distribué à partir du matériau générateur d'aérosol à l'utilisateur ;
dans lequel le comportement d'utilisateur par défaut est déterminé sur une inhalation ; ou
dans lequel la circuiterie de commande est configurée pour déterminer un temps entre l'inhalation et une inhalation suivante sur la base des signaux de détection d'inhalation, et dans lequel la détermination du comportement par défaut de l'utilisateur est également basée sur le temps entre l'inhalation et la prochaine inhalation ; ou
dans lequel le comportement d'utilisateur par défaut est déterminé sur une pluralité d'inhalations, et un temps entre chacune de la pluralité d'inhalations est inférieur à un temps prédéterminé ; ou
dans lequel le comportement d'utilisateur par défaut est déterminé sur une période de temps glissante ;
et **caractérisé en ce que** le seuil est déterminé sur la base du comportement d'utilisateur par défaut déterminé.

12. Support de stockage lisible par ordinateur comprenant des instructions qui, lorsqu'elles sont exécutées par un processeur, réalisent un procédé comprenant :
la réception de signaux de détection d'inhalation en provenance d'un capteur (25) configuré pour détecter une inhalation sur le système de fourniture d'aérosol (10) par un utilisateur du système de fourniture d'aérosol ;
la détermination d'une indication d'une quantité d'un ingrédient distribué à partir d'un matériau (38) générateur d'aérosol aérosolisé par un générateur (36) d'aérosol à l'utilisateur pendant l'inhalation sur la base des signaux de détection d'inhalation ;
la comparaison de l'indication de la quantité de l'ingrédient à un seuil ; et
la fourniture d'une notification à l'utilisateur sur la base de la comparaison entre l'indication de la quantité de l'ingrédient distribué au seuil,
dans lequel le procédé comprend en outre la détermination du comportement d'utilisateur par défaut sur la base de l'indication de la quantité de l'ingrédient distribué à partir du matériau générateur d'aérosol à l'utilisateur ;
dans lequel le comportement d'utilisateur par défaut est déterminé sur une inhalation ; ou
dans lequel la circuiterie de commande est configurée pour déterminer un temps entre l'inhalation et une inhalation suivante sur la base des signaux de détection d'inhalation, et dans lequel la détermination du comportement par défaut de l'utilisateur est également basée sur le temps entre l'inhalation et la prochaine inhalation ; ou
dans lequel le comportement d'utilisateur par défaut est déterminé sur une pluralité d'inhalations, et un temps entre chacune de la pluralité d'inhalations est inférieur à un temps prédéterminé ; ou
dans lequel le comportement d'utilisateur par défaut est déterminé sur une période de temps glissante ;
et **caractérisé en ce que** le seuil est déterminé sur la base du comportement d'utilisateur par défaut déterminé.
